# EUROPEAN PATENT APPLICATION

(11) **EP 1 811 284 A1**
(43) Date of publication of application: **25.07.2007**
(21) Application number: 05800408.6
(22) Date of filing: 07.11.2005
(51) Int. Cl.: G01N 19/00, G01N 33/53

(54) **METHOD OF DETECTING CALMODULIN STRUCTURE CHANGE, METHOD OF RETRIEVING MATTER HAVING ACTIVITY AFFECTING CALMODULIN STRUCTURE CHANGE**

(30) Priority: 09.11.2004 JP 2004325005
(71) Applicant: Fuence Co., Ltd., Tokyo 150-0012 (JP)
(72) Inventor: MOGAMI, Kaname, c/o FUENCE CO., LTD., Shibuya-ku, Tokyo 150-0012 (JP); KASE, Hiroshi, c/o FUENCE CO., LTD., Shibuya-ku, Tokyo 150-0012 (JP)
(74) Representative: HOFFMANN EITLE
(86) International application number: PCT/JP2005/020377
(87) International publication number: WO 2006/051756

(57) **Abstract**

The object of this invention is to provide a method for detecting conformational change of calmodulin easily and simply in a short period, quantitatively and in real-time manner, and to provide a method for screening a substance having an activity that affects to conformational change of calmodulin efficiently among numerous substances. To solve the object, a method is provided and the method comprising; subjecting a test sample to be tested for its activity to a sample film comprising calmodulin, detecting change(s) in tension and/or elasticity of the sample film in comparison with prior to subjecting said test sample using a mechanochemical sensor, thereby it enables real-time and short time measurement of the activity of said test sample to induce or to inhibit conformational change of calmodulin,

## Description

### BACKGROUND OF THE INVENTION

### 1. Field of the Invention

This invention relates to a method for detecting conformational change of calmodulin, a method for screening a substance having an activity that affects to conformational change of calmodulin.

### 2. Related Art

In many intracellular signal transduction systems that utilize calcium as their second messenger, calmodulin binds with calcium at first. Calmodulin is a protein having molecular weight of 167,000 consisting of 148 amino acids, and calmodulin is a calcium binding protein that participates to function of signal transduction. One calmodulin molecule can binds with four calcium molecules. Calmodulin is involved in regulation of various calmodulin dependent enzymes/calmodulin binding substances. At first, calcium binds with calmodulin accompanied with conformational change of the protein, and subsequently, the calmodulin-calcium complex binds with the calmodulin dependent enzyme(s) or the calmodulin binding substance(s), thereby the enzyme(s) or the substance(s) is (are) activated.

So far, calmodulin inhibitors have been screened through measurement of activitie(s) of the calmodulin dependent enzyme(s). This is not a method that determines the effects of a substance toward calmodulin directly, and the activity of calmodulin dependent enzyme(s) is (are) measured indirectly in the presence and absence of calcium/calmodulin, such method is complicated and takes time. Moreover, because the method is an indirect method, it is not admitted that its effect to calmodulin can be measured precisely.

### SUMMARY OF THE INVENTION

Therefore, the object of this invention is to develop a method to evaluate conformational change of calmodulin simply and easily in a short period, by detecting and measuring the process of conformational change of calmodulin using a force sensor at real-time, as change in tension and/or elasticity. Moreover, it is also the object of this invention to enable efficient screening of ligands that affect to conformational change of calmodulin among numerous substances using the method.

The inventors have noticed on tension and/or elasticity change of sample film comprising calmodulin at addition of a substance that affects as a ligand of calmodulin, and made intensive investigations. As the result, the inventors found that conformational change of calmodulin caused by a calmodulin ligand can be detected by measuring the tension and/or elasticity change of the sample file comprising calmodulin using a force sensor. That is, present invention provides a method for detecting conformational change of calmodulin, the method comprising: forming a sample film on a substrate, the sample film comprising calmodulin, placing said substrate comprising said sample film on a force sensor, and; detecting change(s) in tension and/or elasticity caused by conformational change of said sample film when a test sample is subj ected to said sample film, by said force sensor.

Moreover, present invention provides a method for screening a substance having an activity that affects to conformational change of calmodulin, the method comprising: forming a sample film on a substrate, the sample film comprising calmodulin, a fragment of calmodulin, a variant of calmodulin, calmodulin added with a tag, or an antibody protein against calmodulin, placing said substrate comprising said sample film on a force sensor, detecting change(s) in tension and/or elasticity caused by conformational change of said sample film when a test sample is subjected to said sample film, by said force sensor,

Moreover, present invention provides a method for screening a therapeutic or diagnostic agent for diseases that arise change(s) in intracellular signal transduction pathway in which calcium/calmodulin is involved, the method comprising: forming a sample film on a substrate, the sample film comprising calmodulin, a fragment of calmodulin, a variant of calmodulin, calmodulin added with a tag, or an antibody protein against calmodulin, placing said substrate comprising said sample film on a force sensor, and; detecting change(s) in tension and/or elasticity caused by conformational change of said sample film when a test sample is subjected to said sample film, by said force sensor.

This invention provides a method for detecting conformational change of calmodulin, the method comprising; adding a substance (to be tested for its activity) to a sample film in which calmodulin is immobilized, detecting change(s) in tension and/or elasticity of said sample film compared with prior to addition of said substance. According to the method of this invention, the activity of said substance to cause conformational change of calmodulin can be detected efficiently at real-time in a short period.

### BRIEF DESCRIPTION OF THE DRAWINGS

[Fig.1] Fig.1 is a graph showing the effects of CaCl₂ and EDTA on tension and elasticity change of calmodulin (Example 1).
[Fig.2] Fig.2 is a graph showing the effect of calcium ion and calmodulin inhibitor W-7 on tension and elasticity change of calmodulin (Example 2).
[Fig.3] Fig.3 is a graph showing the effect of calmodulin binding site fragment of calcium dependent protein kinase II and calcium ion on tension and elasticity change of calmodulin (Example 3).

### DETAILED EXPLANATION OF THE INVENTION

As described above, present invention provides a method for detecting conformational change of calmodulin, the method comprising; subjecting a substance to be tested to a sample film of calmodulin, and detecting change(s) in tension and/or elasticity of the sample film caused by conformational change of calmodulin by a force sensor. According to the method of this invention, conformational change of calmodulin is directly observed and detected directly as change(s) in tension and/or elasticity at real-time. In this regard, the method of this invention differs from conventional indirect methods of measuring activities of calmodulin dependent enzymes. Moreover, substances having activity to affect to conformational change of calmodulin can be screened using the method of this invention. According to the method of this invention, substances having activity to affect to conformational change of calmodulin can be screened directly, in addition, the time needed for the screening can be shortened significantly, and screening can be conducted efficiently among numerous substances.

Moreover, significant feature of this invention lies in that such conformational change of calmodulin can be detected using change in mechanical property of a sample film comprising calmodulin. According to the method of present invention, the change in mechanical property of sample film can be measured using tension only, elasticity only, otherwise using both of tension and elasticity as the index, present invention is not to be restricted to the particular embodiment described above.

According to present invention, at first, a sample film comprising calmodulin may be prepared on a substrate. The size of the sample film may preferably be 50 to 1000 µm in length, 200 to 2000 µm in width, and 0.3 to 10 µm in thickness, but not limited to these dimensions. Also, the substrate in the present invention may be an appropriate film support, which enables to carry the sample film to a measuring apparatus, by preparing the sample film on the support. The material and size of the substrate is not particularly limited.

It is not requisite to use calmodulin as its intact protein, so long as its function as calmodulin is maintained, a fragment or a variant thereof, or calmodulin added with a tag may be also used. That is, in this specification, "a fragment of calmodulin, a variant of calmodulin, calmodulin added with a tag" means a fragment protein comprising a part of amino acid sequence of calmodulin, a protein in which a part of amino acid sequence of calmodulin is mutated, or a protein to which a tag is added to calmodulin, while maintaining the function as calmodulin, that is, binding with calcium and activating calcium dependent enzyme. In addition, the range of this invention is not limited to the embodiment of forming a sample film comprising calmodulin itself. A film comprising an antibody protein against calmodulin or a protein activated by calmodulin may be formed, and conformational change when calmodulin binds with the antibody or the protein activated by calmodulin may be measured, thereby the same effect can be achieved, therefore, such embodiment is also within the range of this invention.

Meanwhile, as the main examples of calmodulin dependent enzyme/calmodulin binding substance located downstream of the signal transduction pathway mediated by calcium-calmodulin; adenyl cyclase, brushborder myosin I heavy chain, calcineurin, calmodulin dependent protein kinase II, calmodulin dependent protein kinase IV, caldesmon, calmodulin dependent cyclic nucleotide phosphodiesterase, blood cell cyclic-ATPase, neuronal nitric oxide synthase, nicotinamide dinucleotide kinase, phosphatidyl inositol 3 kinase, phosphorylase kinase, skeletal muscle myosin light chain kinase, smooth muscle myosin light chain kinase, IQGAPI can be listed, but not limited to them.

The method of this invention enables screening of ligands that cause conformational change of calmodulin at the condition of only calmodulin, in addition, by binding calmodulin with above-mentioned calmodulin dependent enzyme/calmodulin binding substance, ligands that affect to the calmodulin-calmodulin dependent enzyme complex can be also screened. That is, a sample film comprising calmodulin can be treated with the calmodulin dependent enzyme/calmodulin binding substance to form their complex, otherwise a sample film comprising the calmodulin dependent enzyme/calmodulin binding substance can be treated with calmodulin to form their complex, then screening can be conducted using the complex, which is also one embodiment of this invention. Considering that the calmodulin dependent enzyme/calmodulin binding substance regulates its downstream signal transduction pathway, the embodiment of forming such complex to conduct screening is preferred in this invention.

It is not requisite that this calmodulin dependent enzyme/calmodulin binding substance should be an intact protein, so long as its function as the calmodulin dependent enzyme/calmodulin binding substance can be maintained, a fragment or a variant thereof, or a protein added with a tag may be also used. That is, in the present specification, "a fragment of calmodulin dependent enzyme/calmodulin binding substance, a variant of said protein, said protein added with a tag" means a fragment protein comprising a part of amino acid sequence of above-mentioned calmodulin dependent enzyme/calmodulin binding substance, a protein in which a part of amino acid sequence of the calmodulin dependent enzyme/calmodulin binding substance is mutated, or a protein to which a tag is added to the calmodulin dependent enzyme/calmodulin binding substance, while maintaining the function as calmodulin dependent enzyme/calmodulin binding substance, that is, binding with calmodulin to activate it. In addition, the range of this invention is not limited to the embodiment of immobilizing the calmodulin dependent enzyme/calmodulin binding substance itself. Antibodies against the calmodulin dependent enzyme/calmodulin binding substance can be immobilized and the same effect can be obtained, therefore, such embodiment is also within the range of this invention.

As a means to prepare sample film of calmodulin described above, ESD method (electrospray deposition method), which forms a thin film by depositing the sample by electrospray method is preferred. The technique is known among those skilled in the art, so they can use such techniques for the purpose of this invention with proper modification. As an example of such reference disclosing such a technique, WO 2002-511792 can be listed, which describes a method of producing a deposition of nonvolatile substances including macro-biomolecules using electrospray.

Moreover, Japanese Patent Publication No.2003-136005 describes a device for immobilizing macro-biomolecules and the like to form thin layers and spots while retaining the activities of the biomolecules. Furthermore, WO 2002-503332 describes a method and an apparatus for measuring binding of a ligand to a DNA or a protein. According to the apparatus described in WO 2002-503332, the effect of chemicals to a sample film comprising biomacromolecules can be measured mechanochemically. Therefore, changes in tension and/or elasticity of the sample film can be detected using the apparatus described in WO 2002-503332, as a preferred embodiment of the present invention.

Now, mechanochemical methods for measuring the elasticity of a protein film, to measure the interaction between a ligand and a protein, are described in V.N, Morozov and T.Ya. Morozova (1992) Anal. Biochem., 201:68-79 and in , V.N, Morozov and T.Ya. Morozova (1984) FEBS Letters I75:299-302. Those skilled in the art can achieve appropriate modifications with reference to these documents to carry out the present invention.

Moreover, an intermediate layer comprising water-soluble polymer can be provided between said substrate and said sample film, as a preferred embodiment of the present invention. In the following examples, 1.2% polyvinyl pyrrolidone (PVP) is used as such an intermediate layer. Such an intermediate layer facilitates detachment of the sample film from the substrate. When PVP is used as the intermediate layer, concentration of the PVP may be 0.1 to 5%, preferably 0.3 to 2 %, but the concentration of PVP is not particularly limited. Other water-soluble polymers may be also used.

As described in WO 2002-503332, examples of materials which can be used as this intermediate layer may include: (1) a water-soluble polymer layer, such as polyacrylamide or polyethylene glycol; (2) a layer of polymer having disulfide bonds which can be reduced by mercaptoethanol; (3) a layer of highly dispersed carbon having low adherence to the deposited biomolecules; and (4) a layer of conductive composites of carbonpolymers having low melting point.

If necessary, after immobilization by ESD method, calmodulin comprising said sample film can be further cross-linked. Such cross-linking is not requisite, but it is effective for the purpose to maintain the form and strength of the sample film. Cross-linking reagents available for polymerizing biomolecules are well-known to those skilled in the art. For instance, Hermanson et al., Immobilized Affinity Ligand Techniques Academic Press, New York, 1991 can be used as a reference.

As a reagent used for cross-linking protein, glutaraldehyde, used in the following examples, is the most preferred. Moreover, the reagents for protein cross-linking may include, but are not limited to, zero-length cross-linking reagents such as 1-ethyl-3-(3-dimethylamino) propyl carbodiimide (EDC); homo-bifunctional cross-linking reagents such as dimethyl adipinimidate (DMA); hetero-bifunctional cross linking reagents such as succinimidyl 3-(2-pyridyldithio)propionate (SPDP); and trifunctional cross-linking reagents such as 4-azide-2-nitrophenylbiocytin-4-nitrophenyl ester. Further, the time period for cross-linking reaction is not specifically limited, the optimum condition may be selected accordingly within the range of about 0 to 3 hours.

The sample film thus prepared can be placed to the detecting apparatus described in WO 2002-503332, then immersed into an appropriate buffer solution to prepare for subjecting the a test sample to the sample film, The buffer solution to be used here may include, but not limited to, Hepes buffer and Tris buffer commonly used in this art. The pH of the buffer solution is not particularly limited either. The appropriate pH may be selected accordingly within the range of about pH3 to pH9.

Furthermore, said buffer solution may have an appropriate salt intensity. It is a preferred embodiment of the present invention to add about 0.1 M of sodium chloride to the buffer solution, as described in the following Examples. Measurement can be conducted without adding an electrolyte for giving the salt intensity, and such embodiment is also within the scope of the present invention. Further, the electrolyte to be added is not limited to sodium chloride.

After flowing said buffer solution at a constant flow rate to stabilize the tension of the sample film, said buffer solution may be replaced by a buffer solution containing a test sample which is the target of the assay, and it may subjected to the sample film. The change(s) in tension and/or elasticity of the sample film may be measured before and after addition of the test sample using a force sensor to evaluate their effects on the conformational change of calmodulin. The change(s) in tension and/or elasticity can be measured by a force sensor, preferably by a mechanochemical sensor. The measurement may be conducted by a mechanochemical sensor using an apparatus described in WO 2002-503332, as a particularly preferred embodiment of the present invention.

Furthermore, various substances can be used as the test sample to be examined on their effects to conformational change of calmodulin. The substances which can be used as the test sample may include, but are not limited to, protein, peptide, amino acid, sugar, lipid, nucleic acid, metal and organic compound.

The present invention enables to detect alteration(s) in tension and/or elasticity of calmodulin rapidly at real time. Therefore, conformational change of calmodulin can be evaluated efficiently in many samples, so the time needed for screening substances inhibiting conformational change of calmodulin can be extremely shortened and substances having such activity can be easily selected among massive substances.

Meanwhile, as the examples of diseases in which occurrence of abnormality in calcium-calmodulin signal transduction pathway is suspected at its downstream, schizophrenia, cardiac hypertrophy, defect of memory, hypertension, inflammation, allergy, diabetes mellitus and cancer can be listed. Moreover, it is known that the target of immune-suppressing drug, such as cyclosporine A and tacrolimus, is calcineurin. Therefore, by searching novel calmodulin inhibitors and further investigating on the safety of said inhibitors, it may possibly lead to development of diagnostic and therapeutic agents for these diseases. Thus present invention provides a new way to obtain such useful diagnostic and therapeutic agents.

### EXAMPLES

This invention will be further explained in detail using following examples and drawings, however, these description is not to limit the range of this invention.

### (Example 1)

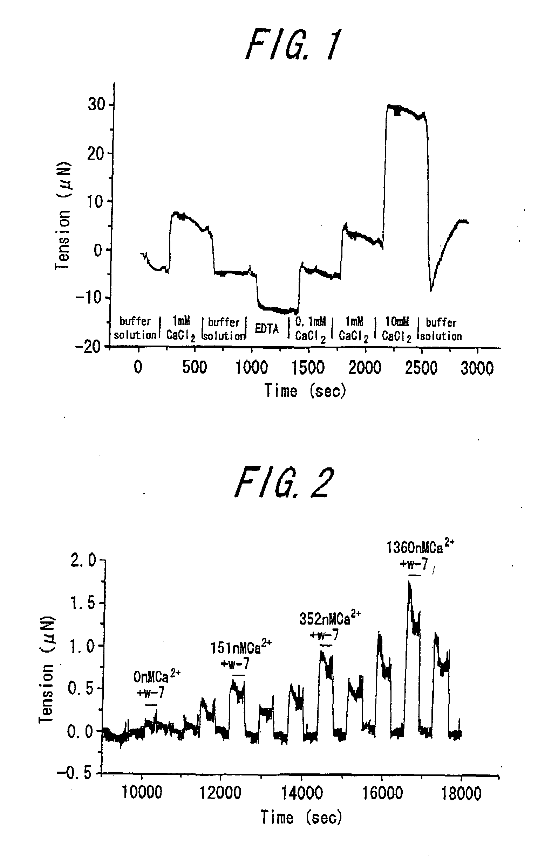

Calmodulin derived from bovine (Sigma) was dissolved into purified water at a concentration of 2mg/ml. This solution was sprayed under dry air using an electrospry device described in WO 2002-511792 or an immobilzing device described in Japanese Patent Publication No.2003-136005. The solution was permeated through a mask with holes of 400 µm in length and 800 µm in width, and then a film was prepared on 1.2% polyvinylpyrrolidone using an electrospray method (the EDS method). The resulting film was placed on an apparatus having a mechanochemical sensor which was described in WO 2002-503332 or U.S. Patent Publication No.6033913, and it was immersed into 10mM Hepes buffer solution pH7.4 (hereinafter referred to as "the buffer solution") containing 0.1 M NaCl. The buffer solution was passed through the film existing on the detecting apparatus at the flow rate of 0.1 to 0.2 mL/min, in order to stabilize the tension of the sample film. Thereafter CaCl₂ solution dissolved in said buffer solution was passed through at the same flow rate to detect changes in tension and elasticity (Fig.1).

This graph indicates that isotropic tension of the calmodulin film remarkably increases by CaCl₂. On the other hand, for the state of the film is restored by the buffer solution to the state prior to addition of CaCI₂, it is indicated that the effect of CaCl₂ to be reversible. Moreover, the result that the tension further decreases by EDTA, a chelator, indicates that a trace amount of Ca ion contained in the buffer solution can be detected. A place where the horizontal line (indicating the initial state) uprises means a point where tension is given to the sample film, and compliance is detected where the line oscillates. This Example shows conformational change of calmodulin caused by Ca²⁺ through interaction between calmodulin and Ca²⁺, which can be detected within several minutes at real-time using present invention. Meanwhile, for example, following review can be refereed to the conformational change of calmodulin, (Vetter S. W. and Leclerc, E. (2003). Novel aspects of calmodulin target recognition and activation. Eur. J. Biochem. 270, 404-414).

### (Example 2)

Calmodulin film, prepared by the same material and by the same method as Example 1, was immersed into 10 mM MOPS buffer solution pH7.2 containing 0.1 M kCl and 10mM EGTA. Calcium buffer solutions with free calcium concentrations of 151nM, 352nM and 1360nM were prepared, then the solutions were flew through without or with addition of 50 µM calmodulin inhibitor W-7, and changes in tension and elasticity were detected (Fig.2).

W-7 caused tension to the calmodulin by itself, and the tension increased in the presence of calcium, but it was restored by removal of W-7. It is known that W-7 binds with calmodulin that received conformational change by calcium, and makes its structure to be compact (Osawa, M. et al., (1999). Evidence for calmodulin inter-domain compaction in solution induced by W-7 binding. FEBS Letters 442, 173-177). Then this Example shows that changes in tension of calmodulin film caused by calmodulin inhibitor corresponds to conformational change of calmodulin, and this system is useful for detection calmodulin inhibitor.

### (Example 3)

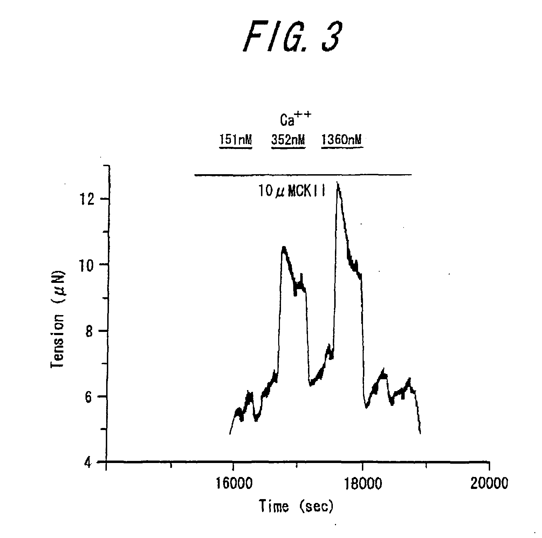

Calmodulin film, prepared by the same material and by the same method as Example 1, was immersed into 10 mM HEPES buffer solution pH7.2 containing 0.1 M kCl and 10mM EGTA. Then a solution containing calmodulin binding site fragment of calmodulin dependent protein kinase II was prepared, it was contacted with calmodulin film and changes in tension and elasticity were detected (Fig.3).

### (Industrial applicability)

According to this invention, a method for detecting conformational change of calmodulin using a mechanochemical sensor was provided. Moreover, using the detection method described above, a method for screening a substance having an activity that affects to conformational change of calmodulin was also provided. It is assumed that the method according to this invention is useful to obtain therapeutic or diagnostic agents for diseases in which signal transduction pathway mediated by calcium-calmodulin is involved.

## Claims

1. A method for detecting conformational change of calmodulin, the method comprising:
forming a sample film on a substrate, the sample film comprising calmodulin, a fragment of calmodulin, a variant of calmodulin, calmodulin added with a tag, or an antibody protein against calmodulin,
placing said substrate comprising said sample film on a force sensor, and;
detecting change(s) in tension and/or elasticity caused by conformational change of said sample film when a test sample is subjected to said sample film, by said force sensor.

2. The method according to Claim 1, wherein said force sensor is a mechanochemical sensor.

3. The method according to Claim 1, wherein the method further comprising the step of binding calmodulin dependent enzyme/calmodulin binding substance, a fragment of said protein, a variant of said protein, said protein added with a tag, or an antibody protein against said protein with said sample film formed on the substrate.

4. The method according to Claim 3, wherein said calmodulin dependent enzyme/calmodulin binding substance is a protein selected from the group consisting of adenyl cyclase, brushborder myosin I heavy chain, calcineurin, calmodulin dependent protein kinase II, calmodulin dependent protein kinase IV, caldesmon, calmodulin dependent cyclic nucleotide phosphodiesterase, blood cell cyclic-ATPase, neuronal nitric oxide synthase, nicotinamide dinucleotide kinase, phosphatidyl inositol 3 kinase, phosphorylase kinase, skeletal muscle myosin light chain kinase, smooth muscle myosin light chain kinase and TQGAPI.

5. A method for screening a substance having an activity that affects to conformational change of calmodulin, the method comprising:
forming a sample film on a substrate, the sample film comprising calmodulin, a fragment of calmodulin, a variant of calmodulin, calmodulin added with a tag, or an antibody protein against calmodulin, placing said substrate comprising said sample film on a force sensor, detecting change(s) in tension and/or elasticity caused by conformational change of said sample film when a test sample is subjected to said sample film, by said force sensor.

6. The method according to Claim 5, wherein said force sensor is a mechanochemical sensor.

7. The method according to Claim 5, wherein the method further comprising the step of binding calmodulin dependent enzyme/calmodulin binding substance, a fragment of said protein, a variant of said protein, said protein added with a tag, or an antibody protein against said protein with said sample film formed on the substrate.

8. The method according to Claim 7, wherein said calmodulin dependent enzyme/calmodulin binding substance is a protein selected from the group consisting of adenyl cyclase, brushborder myosin I heavy chain, calcineurin, calmodulin dependent protein kinase II, calmodulin dependent protein kinase IV, caldesmon, calmodulin dependent cyclic nucleotide phosphodiesterase, blood cell cyclic-ATPase, neuronal nitric oxide synthase, nicotinamide dinucleotide kinase, phosphatidyl inositol 3 kinase, phosphorylase kinase, skeletal muscle myosin light chain kinase, smooth muscle myosin light chain kinase and IQGAPI.

9. A method for screening a therapeutic or diagnostic agent for diseases that arise changes in intracellular signal transduction pathway to which calcium/calmodulin is involved, the method comprising:
forming a sample film on a substrate, the sample film comprising calmodulin, a fragment of calmodulin, a variant of calmodulin, calmodulin added with a tag, or an antibody protein against calmodulin, placing said substrate comprising said sample film on a force sensor, detecting change(s) in tension and/or elasticity caused by conformational change of said sample film when a test sample is subjected to said sample film, by said force sensor.

10. The method according to Claim 9, wherein said force sensor is a mechanochemical sensor.

11. The method according to Claim 9, wherein the method further comprising the step of binding calmodulin dependent enzyme/calmodulin binding substance, a fragment of said protein, a variant of said protein, said protein added with a tag, or an antibody protein against said protein with said sample film formed on the substrate.

12. The method according to Claim 11, wherein said calmodulin dependent enzyme/calmodulin binding substance is a protein selected from the group consisting of adenyl cyclase, brushborder myosin I heavy chain, calcineurin, calmodulin dependent protein kinase II, calmodulin dependent protein kinase IV, caldesmon, calmodulin dependent cyclic nucleotide phosphodiesterase, blood cell cyclic-ATPase, neuronal nitric oxide synthase, nicotinamide dinucleotide kinase, phosphatidyl inositol 3 kinase, phosphorylase kinase, skeletal muscle myosin light chain kinase, smooth muscle myosin light chain kinase and IQGAPI.

13. A sample film comprising calmodulin protein.
